# EUROPEAN PATENT SPECIFICATION

(11) **EP 1 208 852 B1**
(45) Date of publication and mention of the grant of the patent: **01.08.2007**
(21) Application number: 00204183.8
(22) Date of filing: 24.11.2000
(51) Int. Cl.: A61K 51/08, A61P 35/00

(54) **Neuropeptide Y1 receptor binding compounds in the treatment and diagnosis of cancer**
Neuropeptid-Y1-Rezeptor-bindende Verbindungen zur Behandlung und Diagnose von Krebserkrankungen
Composés fixantes de récepteur de Neuropeptide Y1 dans le traitement et le diagnostic du cancer

(43) Date of publication of application: 29.05.2002
(73) Proprietor: Reubi, Jean-Claude, 3084 Wabern (CH)
(72) Inventor: Reubi, Jean-Claude, 3084 Wabern (CH)
(74) Representative: Hooiveld, Arjen Jan Winfried

(56) References cited:
- EP-A- 0 599 303
- EP-A- 0 892 053
- WO-A-00/50086
- HEPPELER A ET AL: "RECEPTOR TARGETING FOR TUMOR LOCALISATION AND THERAPY WITH RADIOPETIDES" CURRENT MEDICINAL CHEMISTRY,BENTHAM SCIENCE PUBLISHERS BV,BE, vol. 7, no. 9, 2000, pages 971-994, XP000982225 ISSN: 0929-8673
- KAZUHIKO TATEMOTO ET AL: "SYNTHESIS OF RECEPTOR ANTAGONISTS OF NEUROPEPTIDE Y" PROCEEDINGS OF THE NATIONAL ACADEMY OF SCIENCES OF USA,US,NATIONAL ACADEMY OF SCIENCE. WASHINGTON, vol. 89, no. 4, 15 February 1992 (1992-02-15), pages 1174-1178, XP000259387 ISSN: 0027-8424
- GEHLERT D R: "SUBTYPES OF RECEPTORS FOR NEUROPEPTIDE Y: IMPLICATIONS FOR THE TARGETING OF THERAPEUTICS" LIFE SCIENCES,GB,PERGAMON PRESS, OXFORD, vol. 55, no. 8, 1994, pages 551-562, XP000612039 ISSN: 0024-3205
- WAHLESTEDT C ET AL: "NEUROPEPTIDE Y-RELATED PEPTIDES AND THEIR RECEPTORS-ARE THE RECEPTORS POTENTIAL THERAPEUTIC DRUG TARGETS?" ANNUAL REVIEW OF PHARMACOLOGY AND TOXICOLOGY,US,ANNUAL REVIEW INC., PALO ALTO, CA, vol. 32, 1993, pages 309-352, XP000612055 ISSN: 0362-1642
- O'SHEA D ET AL: "NEUROPEPTIDE Y INDUCED FEEDING IN THE RAT IS MEDIATED BY A NOVEL RECEPTOR" ENDOCRINOLOGY,US,BALTIMORE, MD, vol. 138, no. 1, 1997, pages 196-202, XP002040613 ISSN: 0013-7227
- AICHER S.A. ET AL: 'RECEPTOR-SELECTIVE ANALOGS DEMONSTRATE NPY/PYY RECEPTOR HETEROGENEITY IN RAT BRAIN' NEUROSCIENCE LETTERS vol. 130, 1991, LIMERICK, IE, pages 32 - 36

## Description

The present invention relates to the diagnosis and treatment of tumors expressing NPY₁ receptors, in particular breast cancer, ovarium cancer and glioblastoma.

NPY is a member of a family of 36 amino acid long peptides, including NPY, peptide YY (PYY) and pancreatic polypeptide (PP). Its main function is a neurotransmitter role, and one of its best known actions in the CNS is stimulation of feeding behavior and inhibition of anxiety. Peripheral actions include effects on gastrointestinal motility and secretion, insulin release, renal secretion and vasoconstriction. The effect of NPY can be mediated by several NPY receptor subtypes, named Y₁-Y₆, from which Y₁, Y₂, Y₄ and Y₅ have been extensively characterized. Several NPY analogs, in particular Y₁ and Y₂ antagonists, are being developed for potential clinical use to treat feeding disturbaces and anxiety. In an article by D. O'Shea et al. (Endocrinology, vol. 138, no.1, 1997, pp 196-202) mention is made of the NPY receptor subtypes Y₁-Y₄ in relation to the stimulation of feeding in animals. A publication of S.A. Aicher et al. (Neuroscience Letters, vol. 130, 1991, pp 32-36) relates to the use of labelled NPY peptide compounds to demonstrate NPY/PYY receptor heterogeneity in rat brains.

Compared to other regulatory peptides, NPY has never been associatated with humen cancer. In the research that led to the present invention, the inventor has investigated whether there was a molecular basis for a putative NPY role in human tumors and/or for the development of NPY drugs for tumor targeting. NPY receptors were evaluated in one of the most frequent and harmful cancers, viz. breast carcinoma. In more than 100 human breast tumor and metastasis samples, the expression of the two best investigated NPY receptor subrtypes, Y₁ and Y₂, was studied using in vitro receptor autoradiography and in situ hybridization.

Based on the high density and high incidence of Y₁, the inventor contemplated that breast cancers represent an important target for NPY-related drugs. It was found that in analogy to other overexpressed peptide receptors, breast tumors can be targeted with radiolabeled Y₁ analogs for diagnostic in vivo scintigraphic tumor detection or for receptor-mediated radiotherapeutic treatment of these tumors in a similar manner as described for somatostatin (Colmers & Bleakman, Trends Neurosci. 17, 1994, pp 373-379; Wettstein et al., Pharmacol. Ther. 65,1995, pp 397-414).
A. Heppeler et al. (Current Med. Chem.. vol. 7,200, pp 971-994) have written a review article about receptor targeting for tumor localisation and therapy with radiolabeled peptides, viz. labeled analogs of somatostatin, α-MSH, neurotensin, VIP, bombesin, substance P, and CCK. This review is completely silent on NPY in relation to tumors. The same holds for WO 0050086A (Mallinckrodt Inc., US), of August 31, 2000, wherein no additional information to the review article of Heppeler et al. can be found. These known peptide compounds can be used for targeting certain specific receptors or receptor subtypes. Numerous different human malignant tumor types have been found, all expressing their own specific receptors or receptor subtypes. In the present case the inventor has found that certain malignant tumors express a specific receptor subtype, viz. neuropeptide Y₁, and that normal adjacent tissue does not express this receptor subtype, offering the possibility to selectively target these tumors.
Moreover, long-term treatment with Y₁-selective analogs (Hökfelt et al., Neuropharmacology 39, 2000, pp 1337-1356; J.H. Walsh: "Gastrin. In Gut Peptides": Biochemistry and Physiology, pp 75-121 (eds. J.H. Walsh and G.J. Dockray; Raven Press, New York, 1994)) is of considerable interest when NPY affects the proliferation of tumors.

It was found according to the present invention, that the neuropeptide Y₁ receptor is exclusively expressed on tumor tissue either in combination with the Y₂ receptor or alone, whereas healthy tissue only expresses the Y₂ receptor.

*The present invention relates to the use of a neuropeptide Y₁ (NPY₁) receptor binding peptide compound, selected from the group consisting of [Leu³¹, Pro³⁴]-NPY, [Leu³¹, Pro³⁴]-PYY, Pro³⁴-PYY, NPY, PYY, Des Asn²⁹[Trp^{28,32}, Nva³⁴]-NPY(27-36), [Pro³⁰, Tyr³², Leu³⁴]-NPY(28-36), the dimer Bis (31*/*31'){[Cys³¹, Trp³², Nva^{J4}]-NPY(31-36)}, SR120819A, BIBP3236,*
*the compound 383 U91 of the formula* 
IleCysProCysTyrArgLeuArgTyrNH2
|          |
H2NTyrArgLeuArgTyrCysProCysIle,
 *compound 1120W91 of the formula* 
IleAsnProDpaTyrArgLeuArgTyrNH2
|
IleAsnProDpaTyrArgLeuArgTyrNH2,
 *compound 1229U91 of the formula* 
IleGluProDprTyrArgLeuArgTyrNH2
|          |
H2NTyrArgLeuArgTyrDprProGluIle
 *which compounds show a high receptor binding affinity in displacing a NPY₁-selective radioligand in displacing experiments.*
*This high receptor binding affinity has been shown in experiments (see the Example), wherein displacement of 25 nM of the radioligand ¹²⁵I-PYY in the presence of 25 nM of*
*said peptide compound is shown in autoradiograms of hematoxylin-eosin stained breast tumor tissue but not in adjacent normal breast tissue.*

Targeting these tumors is a powerful tool, not only in diagnosing such tumors but also in supporting an effective therapy. As a matter of fact, in order to be able to achieve a specific therapy for the control of such tumors, the detection and localization of these tumors, and in particular of the metastases thereof, in an early stage of their development is of utmost importance. Various requirements have to be imposed on an agent that is used in such a diagnostic method, such as non-toxic, no adverse influence on the host resistance and/or on the therapeutic treatment, well detectable and highly selective. The required high selectivity means that the diagnostic agent, after having been introduced into the body, must accumulate more strongly in the target tumors to be detected or visualized than in surrounding tissues. This selectivity, i.e. a comparatively stronger concentration of the diagnostic agent in the target tumors compared with non-target tissues, enables the user to correctly diagnose the malignancy. In order to be detectable from outside the body, the diagnostic agent should be labeled, preferably with a radionuclide or with a paramagnetic metal atom. In the former case, the radioactive radiation can be detected by using a suitable detector (scanning). Modern techniques in this field use emission tomography; when gamma radiating isotopes are used, the so-called single photon emission computerized tomography (SPECT) may be applied. The use of paramagnetic diagnostic agents enables a detection by means of imaging by magnetic resonance.

For diagnosis, the compound binding the NPY₁ receptor as described above is thus labeled with one of the following markers:
(a) a radioactive metal isotope selected from the group consisting of ^{99m}Tc, ²⁰³Pb, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, ¹⁷⁷Lu and ⁵¹Cr, or
(b) with a paramagnetic metal atom selected from the group consisting of Cr, Mn, Fe, Co, Ni, Cu, Pr, Nd, Sm, Yb, Gd, Tb, Dy, Ho and Er, or
(c) with a radioactive halogen isotope, selected from ¹²³I, ¹³¹I, ⁷⁵Br, ⁷⁶Br, ⁷⁷Br and ⁸²Br.

The thus labeled compound is administered to the subject to be diagnosed in an amount sufficient to be visualized by external imaging, by radioactive scanning or by magnetic resonance imaging, to determine the targeted sites in the body of said being in relation to the background activity, in order to allow detection and localization of the tumors in the body. Such amount will usually vary between 1 and 20 µg.

For treatment, the compound that binds the NPY₁ receptor as described above per se may have a therapeutic effect, or may be coupled to a radioisotope, selected from the group consisting of ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I and ¹³¹I. The compound per se or the compound thus labeled is administered to the subject to be treated in an amount sufficient to be effective. Such amount lies usually between 20 and 1000 µg.

Furthermore, the present invention relates to pharmaceutical compositions for the diagnosis or treatment of breast cancer, in particular in humans, comprising one or more compounds that bind the neuropeptide Y₁ receptor as described above and a suitable carrier, diluent or excipient.

It is frequently impossible to put a ready-for-use pharmaceutical composition at the disposal of the user, especially in the case of radiolabeled compounds due the often poor shelf life thereof and/or the short half-life of the radionuclide used. In such cases the user will carry out the labeling reaction with the radionuclide in the clinical hospital or laboratory. For this purpose the various reaction ingredients are then offered to the user in the form of a so-called "kit". It will be obvious that the manipulations necessary to perform the desired reaction should be as simple as possible to enable the user to prepare from the kit the radioactive labeled composition by using the facilities that are at his disposal. Therefore the invention also relates to a kit for preparing a radiopharmaceutical composition.

Such a kit according to the present invention for preparing a radiopharmaceutical composition comprises (i) a NPY₁ receptor binding compound as defined above, to which compound, if desired, an inert pharmaceutically acceptable carrier and/or formulating agents and/or adjuvants is/are added, (ii) a solution of a salt or chelate of a metal isotope selected from the group consisting of ²⁰³Pb, ⁶⁷Ga, ⁶⁸Ga, ⁷²As, ¹¹¹In, ^{113m}In, ⁹⁷Ru, ⁶²Cu, ^{99m}Tc, ¹⁸⁶Re, ¹⁸⁸Re, ⁶⁴Cu, ⁵²Fe, ^{52m}Mn, ⁵¹Cr, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁶⁵Dy, ¹⁴⁹Pm, , ¹⁵¹Pm, ¹⁵³Sm, ¹⁵⁷Gd, ¹⁶⁶Ho, ¹⁷²Tm, ¹⁶⁹Yb, ¹⁷⁵Yb, ¹⁷⁷Lu, , ¹⁰⁵Rh and ¹¹¹Ag, and (iii) instructions for use with a prescription for reacting the ingredients present in the kit.

Preferably, when the compound is a peptide, the peptide compound to be used as an ingredient of the above kit has been derivatized by a reaction with a chelating agent.

Suitable chelating groups for chelating metal atoms are NₜS₍₄₋ₜ₎ tetradentate chelating agents, wherein t=2-4, or groups derived from ethylene diamine tetra-acetic acid (EDTA), diethylene triamine penta-acetic acid (DTPA), cyclohexyl 1,2-diamine tetra-acetic acid (CDTA), ethyleneglycol-0,0'-bis(2-aminoethyl)-N,N,N',N'-tetra-acetic acid (EGTA), N,N-bis(hydroxybenzyl)-ethylenediamine-N,N'-diacetic acid (HBED), triethylene tetramine hexa-acetic acid (TTHA), 1,4,7,10-tetraazacyclododecane-N,N',N'',N'''-tetra-acetic acid (DOTA), hydroxyethyl-diamine triacetic acid (HEDTA), 1,4,8,11-tetra-azacyclo-tetradecane-N,N',N'',N'''-tetra-acetic acid (TETA), substituted DTPA, substituted EDTA, or from a compound of the general formula 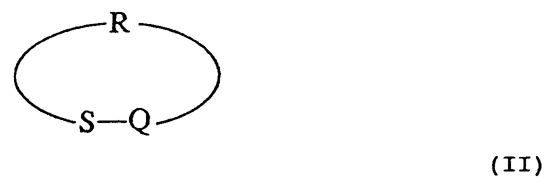 wherein R is a branched or non-branched, optionally substituted hydrocarbyl radical, which may be interrupted by one or more hetero-atoms selected from N, O and S and/or by one or more NH groups, and

Q is a group which is capable of reacting with an amino group of the peptide and which is preferably selected from the group consisting of carbonyl, carbimidoyl, N-(C₁-C₆)alkylcarbimidoyl, N-hydroxycarbimidoyl and N-(C₁-C₆)alkoxycarbimidoyl.

NₜS₍₄₋ₜ₎ chelating agents, wherein t=2-4, are preferably selected from 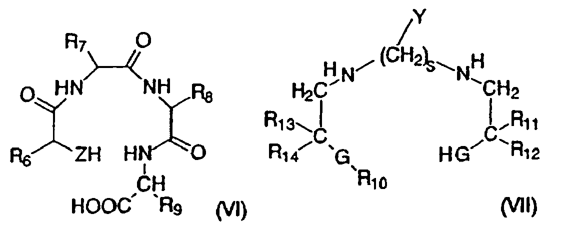 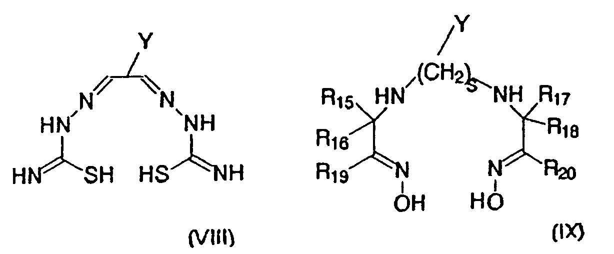 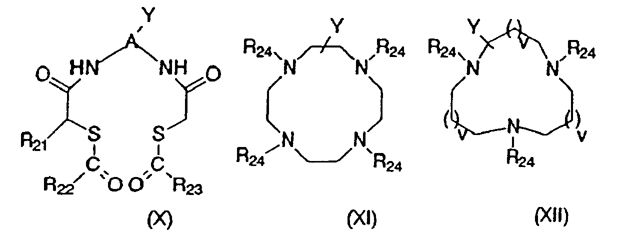 wherein:
R₆-R₂₀ are each individually hydrogen atoms or (C₁-C₄)alkyl groups, with the proviso that at least one of C₆ to C₉ is the symbol Y;
R₂₁ is a hydrogen atom or a CO₂(C₁-C₄)alkyl group;
R₂₂ and R₂₃ are each individually (C₁-C₄)alkyl groups or phenyl groups;
v is 0 or 1;
s is 2 or 3;
R₂₄ is CH₂COOH or a functional derivative thereof;
A is (C₁-C₄)alkylene, if desired substituted with CO₂alkyl, CH₂COalkyl, CONH₂, CONHCH₂CO₂alkyl; phenylene, phenylene substituted by CO₂alkyl, wherein the alkyl groups have 1 to 4 carbon atoms;
G is NH or S;
Y is a functional group capable of binding with a free amino group of the peptide or with the spacing group;
and Z is S or O.
Said functional group Y preferably comprises isocyanato, isothiocyanato, formyl, o-halonitrophenyl, diazonium, epoxy, trichloro-s-triazinyl, ethyleneimino, chlorosulfonyl, alkoxycarb-imidoyl, (substituted or unsubstituted) alkylcarbonyloxycarbonyl, alkylcarbonylimidazolyl, succinimido-oxycarbonyl; said group being attached to a (C₁-C₁₀) hydrocarbon biradical.

Suitable examples of hydrocarbon biradicals are biradicals derived from benzene, (C₁-C₆)alkanes, (C₂-C₆)alkenes and (C₁-C₄) -alkylbenzenes.

Examples of suitable chelators of the general formula II are described in the international patent application WO 89/07456, such as unsubstituted or substituted 2-imino-thiolanes and 2-imino-thiacyclohexanes, in particular 2-imino-4-mercaptomethylthiolane.

Suitable examples of spacing groups, if present in the metal-labeled peptide molecule, are groups of the general formula 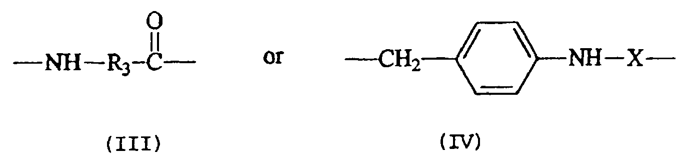 wherein R₃ is a C₁-C₁₀ alkylene group, a C₁-C₁₀ alkylidene group or a C₂-C₁₀ alkenylene group, and X is a thiocarbonyl group or a group of the general formula 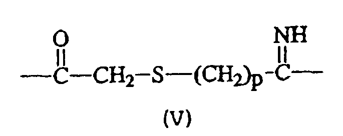 wherein **p** is 1-5.

Alternatively, peptide conjugates with avidin or biotin are formed, for example as described by Paganelli et al. (Int. J. Cancer 1988, 2, 121), Kalofonos et al. (J. Nucl. Med. 1990, 31, 1791) and Anderson et al. (FEBS LETT. 1991, 282/1, 35-40).

The resulting peptide conjugates provide a facility for firmly attaching the radionuclide in a simple manner. Suitable chelating agents for modifying peptides are described in detail hereinbefore. N-containing di- or polyacetic acids or their derivatives, such as the compounds mentioned before, have proved to be pre-eminently suitable for attaching various metal radionuclides, such as ¹¹¹In and ^{113m}In, to peptide molecules. The kit to be supplied to the user may also comprise the ingredient(s) defined sub (i) above, together with instructions for use, whereas the solution of a salt or chelate of the radionuclide, defined sub (ii) above, which solution has a limited shelf life, may be put to the disposal of the user separately.

In case the kit serves to prepare a radiopharmaceutical composition labeled with ^{99m}Tc, ¹⁸⁶Re or ¹⁸⁸Re, such a kit according to the present invention may comprise, in addition to the ingredient(s) defined sub (i) above, (ii) a reducing agent and, if desired, a chelator, and (iii) instructions for use with a prescription for reacting the ingredients of the kit with ^{99m}Tc in the form of a pertechnetate solution, or with ¹⁸⁶Re or ¹⁸⁸Re in the form of a perrhenate solution. If desired, the ingredients of the kit may be combined, provided they are compatible. The kit should comprise a reducing agent to reduce the pertechnetate or perrhenate, for example, a dithionite, a metallic reducing agent or a complex-stabilizing reducing agent, e.g. SnCl₂, Sn(II)-tartrate, Sn(II)-phosphonate or -pyrophosphate, or Sn(II)-glucoheptonate. The pertechnetate or perrhenate solution can simply be obtained by the user from a suitable generator.

When the radionuclide is present in the kit itself, the complex forming reaction with the derivatized peptide can simply be produced by combining the components in a neutral medium and causing them to react. For that purpose the radionuclide may be presented to the derivatized peptide in the form of a chelate bound to a comparatively weak chelator, as described hereinbefore.

When the kit comprises a derivatized peptide as defined hereinbefore and is intended for the preparation of a radiopharmaceutical composition, labeled with ^{99m}Tc, ¹⁸⁶Re or ¹⁸⁸Re, the radionuclide will preferably be added separately in the form of a pertechnetate or perrhenate solution. In that case the kit will comprise a suitable reducing agent and, if desired, a chelator, the former to reduce the pertechnetate or the perrhenate. As a reducing agent may be used, for example, a dithionite or a metallic reducing agent. The ingredients may optionally be combined, provided they are compatible.

Such a monocomponent kit, in which the combined ingredients are preferably lyophilized, is excellently suitable for being reacted, by the user, with the radionuclide solution. As a reducing agent for the above-mentioned kits is preferably used a metallic reducing agent, for example, Sn(II), Ce(III), Fe(II), Cu(I), Ti(III) or Sb(III); Sn(II) is excellently suitable.

The peptide constituent of the above-mentioned kits, i.e. preferably the derivatized peptide, may be supplied as a solution, for example, in the form of a physiological saline solution, or in some buffer solution, but is preferably present in a dry condition, for example, in the lyophilized condition. When used as a component for an injection liquid it should be sterile, in which, when the constituent is in the dry state, the user should preferably use a sterile physiological saline solution as a solvent. If desired, the above-mentioned constituent may be stabilized in the conventional manner with suitable stabilizers, for example, ascorbic acid, gentisic acid or salts of these acids, or it may comprise other auxiliary agents, for example fillers, such as glucose, lactose, mannitol.

The amount of the NPY₁ receptor binding compound in a pharmaceutical composition for diagnosis can be 1 to 20 µg. The composition can be administered through parenteral routes. The amount of the compound to be administered to the patient to be diagnosed is 1 to 20 µg.

The amount of the NPY₁ receptor binding compound in a therapeutic composition can be 20 to 1000 µg. The composition can be administered through parenteral routes. The amount of the compound to be administered to the patient to be treated is 20 to 1000 µg.

The present invention further relates to the use of a composition comprising, in a quantity sufficient for external imaging, a labeled NPY₁ receptor binding peptide compound, as decribed hereinbefore, for the manufacture of a diagnostic agent for detecting and localizing NPY₁ receptor expressing tumors and their metastases in tissues, which in healthy condition do not contain NPY₁ receptors, in the body of a human being, by administering said composition to the being to be diagnosed in an amount of 1 to 20 µg and by radioactive scanning or by magnetic resonance imaging, to determine the targeted sites in the body of said being in relation to the background activity, in order to allow detection and localization of the tumor(s) in the body. Said peptide compound is labelled with (a) a radioactive metal isotope selected from the group consisting of ^{99m}Tc, ²⁰³Pb, ⁶⁷Ga, ⁶⁸ Ga, ⁷²As, ¹¹¹In, ^{113m}In, ⁹⁷Ru, ⁶²Cu, ⁶⁴Cu, ⁵²Fe, ^{52m}Mn, and ⁵¹Cr, or (b) a paramagnetic metal atom selected from the group consisting of Cr, Mn, Fe, Co, Ni, Cu, Pr, Nd, Sm, Yb, Gd, Tb, Dy, Ho and Er, or (c) a radioactive halogen isotope selected from ¹²³I, ¹³¹I, ⁷⁵Br, ⁷⁶Br, ⁷⁷Br and ⁸²Br.

The present invention also relates to the use of a composition comprising, in a quantity sufficient for detection by a gamma detecting probe, a labeled NPY₁ receptor binding peptide compound, as described hereinbefore, for the manufacture of a radioimmunodetection agent for intraoperatively detecting and localizing NPY₁ receptor expressing tumors and their metastases in tissues, which in healthy condition do not contain NPY₁ receptors, in the body of a human being, by administering said composition to the being to be diagnosed in an amount of 1 to 20 µg to determine the targeted sites in the body of said being, in order to allow detection and localization of the tumor(s) in the body. Said peptide compound is labelled with a radioactive isotope selected from ¹⁶¹Tb, ¹²³I or ¹²⁵I.

The present invention finally relates to the use of a composition comprising, in a quantity effective for combating or controlling tumors, a NPY₁ receptors binding peptide compound, as described hereinbefore, for the manufacture of a therapeutic agent for combating or controlling tumors and their metastases in tissues, which in healthy condition do not contain NPY₁ receptors, in the body of a human being, by administering said composition to the being to be treated in an amount of 20 to 1000 µg. Said peptide compound is labelled with an isotope selected from the group consisting of ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I, and ¹³¹I.

Preferably the tumors to be detected, localized or therapeutically treated are selected from the group consisting of breast cancer, ovarian cancer and glioblastoma, and their metastases.

### EXAMPLE

In the example reference is made to the following figures:

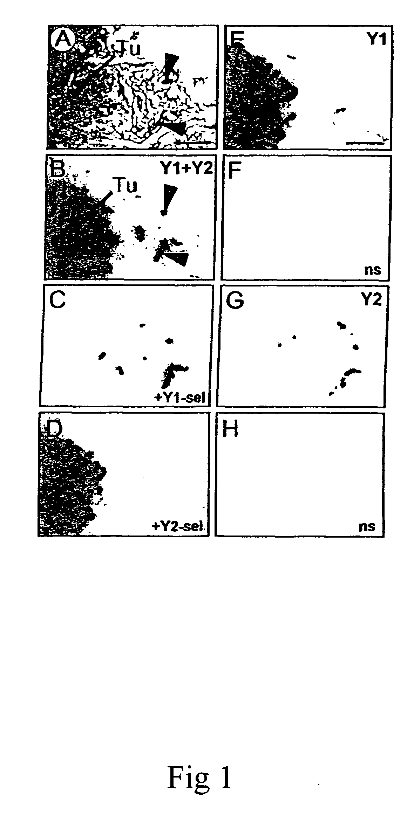

**Fig. 1** NPY receptors in breast carcinoma and adjacent normal breast.
   A: Hematoxylin-eosin staining showing tumor (Tu) and normal breast (arrowheads). Bar = 1 mm.
   B: Autoradiogram showing total binding of ¹²⁵I-PYY with strong labeling of tumor and breast.
   C: Autoradiogram showing ¹²⁵I-PYY binding in presence of 25 nM of the Y₁-selective [Leu³¹, Pro³⁴]-NPY. Complete displacement of the radioligand is seen in tumor but not in breast.
   D: Autoradiogram showing ¹²⁵I-PYY binding in presence of 25 nM of the Y₂-selective PYY(3-36). Complete displacement is seen in breast but not in tumor.
   E: Autoradiogram showing total binding of the Y₁-selective radioligand ¹²⁵I-[Leu³¹, Pro³⁴]-PYY. The tumor is strongly labeled, the breast tissue is not or only very weakly visible.
   F: Autoradiogram showing non-specific binding ¹²⁵I-[Leu³¹, Pro³⁴]-PYY (in presence of 25 nM [Leu³¹, Pro³⁴]-NPY).
   G: Autoradiogram showing total binding of the Y₂-selective radioligand ¹²⁵I-PYY(3-36). Tumor is not seen but adjacent breast tissue is labeled.
   H: Autoradiogram showing non-specific binding of ¹²⁵I-PYY(3-36) (in presence of 25 nM of PYY(3-36)).
**Fig. 2** Competition curves showing Y₁ in human breast carcinoma. The graph shows high affinity displacement of ¹²⁵I-PYY by PYY, [Leu³¹, Pro³⁴]-NPY, and [Leu³¹, Pro³⁴]-PYY, but not by PYY (3-36). Somatostatin (SS-14) is inactive.
**Fig. 3** Y₁ mRNA detected by in situ hybridization in an Y₁-expressing breast tumor.
   A: Hematoxylin-eosin stained section showing breast carcinoma. Bar = 1mm.
   B: Autoradiogram showing Y₁ mRNA in the tumor tissue. Non-specific labeling (in presence of a 20 fold excess of the corresponding probe) is negligible.
   C: Autoradiogram showing binding of I-[Leu³¹, Pro³⁴]-PYY in the same tumor tissue.

### Introduction

In this example it is investigated whether receptors for neuropeptide Y (NPY), a neurotransmitter with yet no established link to human cancer, can be overexpressed in human tumors. More than 100 samples of human breast carcinomas and adjacent breast tissues were tested for their expression of the NPY receptor subtypes Y₁ and Y₂ using in vitro receptor autoradiography with universal as well as subtype-selective analogs and in situ hybridization of Y₁ and Y₂ mRNA.

### Materials and Methods

### Patient tissues

Breast tissue samples with primary breast neoplasias were obtained from 95 patients, aged 36 to 91, who were operated on in several institutions. Tissue samples were kept frozen at -80°C. The diagnosis was reviewed and formulated by use of cryostat sections, according to the WHO guidelines stated by Tavassoli (General considerations. in Pathology of the breast 1st edn (ed. Tavassoli, F.A.) 25-62 (Appleton & Lange, Norwalk, 1992)). In the main group of 89 patients, 61 (69%) showed an invasive ductal carcinoma.

In an additional group of six patients (4 ductal and 2 lobular breast carcinomas), tissue samples obtained from the primary tumor and from all the axillary metastases were investigated.

### NPY receptor autoradiography

Twenty-µm thick cryostat sections of the tissue samples were processed for NPY receptor autoradiography as described in detail previously for other peptide receptors (Reubi, J.C. et al. Cancer Res. 50,5969-5977 (1990)). One radioligand used was ¹²⁵I-PYY (2' 000 Ci/mmol; Anawa, Wangen, Switzerland), known to specifically label NPY receptors. For autoradiography, tissue sections were mounted on precleaned microscope slides and stored at -20°C for at least 3 days to improve adhesion of the tissue to the slide. Sections were then processed according to Dumont et al. (J. Neurosci. 13, 73-86 (1993)). They were first preincubated in 119 mM NaCl, 3.2 mM KCl, 1.19 mM KH₂PO₄, 1.19 mM MgSO₄, 25 mM NaHCO₃, 2.53 mM CaCl₂x2H₂O and 10 mM D-glucose, pH 7.4 (preincubation solution), for 60 min at room temperature. The slides were then incubated in a solution containing the same medium as the preincubation solution in which the following compounds were added: 0.1% bovine serum albumin, 0.05% bacitracin, pH 7.4, and the radioligand, at approximate concentration of 22 pM ¹²⁵I-PYY. The slides were incubated at room temperature with the radioligand for 120 min.

To estimate nonspecific binding, paired serial sections were incubated as described above, except that 25 nM PYY was added to the medium. In order to distinguish Y₁ from Y₂ subtypes, increasing amounts of nonradioactive NPY, [Leu³¹, Pro³⁴]-NPY, a Y₁-selective ligand, and PYY(3-36), a Y₂-selective ligand, were added to the incubation medium to generate competitive inhibition curves using successive sections. Additional analogs used in competition experiments included pancreatic polypeptide and PYY(13-36). In selected cases, displacements with a single concentration (25 nM) of each of the above mentioned peptides were performed.

On completion of the incubation, the slides were washed four times for 5 min each in ice-cold preincubation solution, pH 7.4. They were rinsed twice in ice-cold distilled water, and then dried under a stream of cold air at 4°C, apposed to ³H-Hyperfilms and exposed for 7 days in x-ray cassettes.

In order to further distinguish Y₁ from Y₂ receptors, all cases demonstrating binding with ¹²⁵I-PYY were evaluated with the Y₁-selective radioligand ¹²⁵I-[Leu³¹, Pro³⁴]-PYY and with the Y₂-selective ¹²⁵I-PYY(3-36) (Gehlert et al., Neurochem. Int. 21, 45-67 (1992); Gehlert & Gackenheimer Neurosci. 76, 215-224 (1997)). Identical experimental conditions as mentioned for ¹²⁵I-PYY were used.

The autoradiograms were quantified using a computer-assisted image processing system, as described previously (Reubi et al. (1990), supra; Markwalder & Reubi Can. Res. 59,1152-1159 (1999)). Tissue standards for iodinated compounds (Amersham) were used for this purpose. A tissue was defined as receptor-positive when the optical density measured in the total binding section was at least twice that of the nonspecific binding section (in the presence of 10⁻⁷ mol/L PYY).

In each experiments, Y₁ expressing tissue (rat cortex) and Y₂ expressing tissues (stratum oriens and radiatum of the rat hippocampus) (Dumont et al. (1993), supra) were included as positive controls.

### In situ hybridization histochemistry

Y₁ and Y₂ receptor mRNA were identified in selected normal and tumoral breast tissue samples with in situ hybridization histochemistry on cryostat sections, as described in detail previously (Reubi et al., Cancer Res 54, 3455-3459 (1994)). Oligonucleotide probes complementary to nucleotides 493-529 or 850-879 (Malmström, R.E. et al., Regul. Pept. 75-76, 55-70 (1998)) of the human Y₁ receptor gene and to nucleotides 223-252 (Malmström et al. (1998), supra) of the human Y₂ receptor gene or 1008-1052 (Schwarzer et al., Mol. Pharmacol. 55, 6-13 (1998)) of the rat Y₂ receptor gene (that sequence having 96% homology to the corresponding human one) were synthesized and purified on a 20% polyacrylamide-8M urea sequencing gel (Microsynth, Balgach, Switzerland). They were labeled at the 3'-end by using [α-³²P] dATP (>3,000 Ci/mmol [>111 TBq/mmol]; NEN, Life Science Products, Boston, MA) and terminal deoxynucleotidyltransferase (Boehringer, Mannheim, Germany) to specific activities of 0.9-2.0 x 10⁻⁴ Ci/mmol [33.3-74 GBq/mmol]. Control experiments were carried out as reported previously (Reubi et al. (1994), supra) with the probes used in the present study to determine the specificity of the hybridization signal obtained.

### Results

**Table 1** summarizes the NPY receptor incidence in breast tissue in the main group of 89 patients. NPY receptors were expressed in a total of 76 of the 89 breast carcinomas tested. NPY receptors were found in 58/66 tested ductal carcinomas (55/61 invasive, 3/5 in situ) and 13/15 of the tested lobular carcinomas. Of the special types of invasive carcinomas, 2/3 mucinous carcinomas, 2/2 medullary, 1/1 tubular and 0/2 apocrine carcinomas were NPY receptor-positive.

For Y₁ and Y₂ subtype characterization, the two approaches used in the present study, namely the use of ¹²⁵I-PYY and its displacement by unlabeled Y₁ and Y₂ subtype-selective analogs (Dumont et al. (1993), supra) or the use of the two Y₁- and Y₂-selective radioligands (Gehlert & Gackenheimer (1997), supra) gave congruent results: Y₁ was the predominantly expressed receptor subtype in NPY receptor-positive tumors, with 100% incidence of this subtype in receptor-positive tumors, while Y₂ was only found in 24% of the cases (**Table 1**).

**Table 1**

| Incidence of NPY receptors Y₁ and Y₂ in human breast tissue | | | |
|---|---|---|---|
| Tissue | NPY-R Incidence* | Differentiation by Y₁ and/or Y₂ expression** | Cases with focal R distribution |
| breast carcinomas | 76/89 (85%) | "Y₁-type of tumor***: 58/76 (76%) "mixed Y₁/Y₂" tumor type: 18/76 (24%) "Y₂-type" of tumor: 0/76 (0%) | 21/58 (36%) Y₁: 3/18 (17%) Y₂: 10/18 (55%) |
| non-neoplastic breast (ducts+lobules) | 45/45 (100%) | "Y₂-only" breast type: 19/45 (42%) "switch Y₂/Y₁" breast type: 26/45 (58%) "Y₁-only" breast type: 0/45 (0%) | |

| | | | |
|---|---|---|---|
| * detected with "universal" ligand ¹²⁵I-PYY ** detected with Y₁-selective (¹²⁵I-[Leu₃₁, Pro₃₄]-PYY) or Y₂-selective (¹²⁵I-PYY(3,36)) radioligands *** "Y₁-type" of tumor was defined as those tumors expressing predominantly Y₁ but not Y₂ 46 cases had only Y₁; the remaining 12 cases had a density of Y₂ amounting less than 10% of the Y₁ density. | | | |

In 58/76 (76%) of the receptor-positive tumors, Y₁ was found to be the only (46 cases) or the predominant (12 cases containing more than 90% of Y₁ compared to Y₂) receptor subtype expressed, while in 24% both Y₁ and Y₂ were highly expressed, and in none of the tumors, Y₂ was found alone.

It was noticed that Y₁ was more often homogeneously distributed within the tumor than it was the case for the Y₂ receptor, which was expressed focally, i.e. in certain restricted areas of the tumor only, in 55% of the cases. Even in tumors expressing concomitantly Y₁ and Y₂, no tumor area was found that expressed Y₂ alone; Y₂ was only found in areas where Y₁ was expressed.

In an additional group of six patients not listed in **Table 1**, from whom primary breast tumors could be obtained together with all lymph node metastases, it was identified that the six primaries as well as all the metastases were expressing NPY receptors (**Table 2**); Y₁ was present in all cases, Y₂ in a few cases only, both in primaries as wells as in metastases **(Table 2).**

**Table 2**

| | Primary tumor | | Metastases | | |
|---|---|---|---|---|---|
| | NPY receptor subtype density (dpm/mg tissue) | | | | |
| | **Y₁** | **Y₂** | | **Y₁** | **Y₂** |
| Case 1 (ductal Ca) | 7200 | -- | Meta 1 | 4511 | -- |
| | | | Meta 2 Meta 2 | 2005 | -- |
| | | | Meta 3 | 1420 | -- |
| | | | Meta 4 | 3179 | -- |
| | | | Meta5 5 | 4241 | -- |
| Case 2 (lobular Ca) | 1398 | -- | Meta 1 | 5388 | -- |
| | | | Meta 2 | 5481 | -- |
| | | | Meta 3 | 5418 | -- |
| Case 3 (ductal Ca) | 12262 | -- | Meta 1 | 9430 | -- |
| | | | Meta 2 | 10138 | -- |
| | | | Meta 3 | 12298 | -- |
| | | | Meta 4 | 11041 | -- |
| | | | Meta 5 | 10471 | -- |
| | | | Meta 6 | 11844 | -- |
| | | | Meta 7 | 11015 | -- |
| | | | Meta 8 | 12366 | -- |
| | | | Meta 9 | 9553 | -- |
| Case 4 (ductal Ca | 12542 | 6535 | Meta 1 | 12205 | -- |
| | | | Meta 2 | 9970 | -- |
| | | | Meta 3 | 12102 | 8220 |
| | | | Meta 4 | 12278 | -- |
| | | | Meta 5 | 11060 | 6901 |
| | | | Meta 6 | 11062 | 6957 |
| Case 5 (lobular Ca) | 9787 | 2879 | Meta 1 | 7195 | 3440 |
| | | | Meta 2 | 8512 | 4105 |
| Case 6 (ductal Ca) | 9445 | -- | Meta | 8627 | 4561 |

**Table 1** further shows that NPY receptors can be detected in all tested normal breast tissues as well. In 42% of the cases, the Y₂ receptor is expressed alone, whereas in none of the tested breast tissues the Y₁ receptor is expressed alone. However, in the remaining breast tissues, Y₁ and Y₂ can be expressed concomitantly (58%).

**Fig. 1** is a typical and representative example of the NPY receptor expression in a sample containing a breast carcinoma surrounded by normal breast tissue. The breast carcinoma expresses Y₁ receptors only as shown by the labeling of the tumor by ¹²⁵I-PYY and its displacement with [Leu³¹, Pro³⁴]-NPY but not by PYY(3-36). These results are further confirmed by the additional experiments using two other radioligands: the tumor is labeled by the Y₁-selective ¹²⁵I-[Leu³¹, Pro³⁴]-PYY but not by the Y₂-selective ¹²⁵I-PYY(3-36). Conversely, in the same tissue sections, the surrounding breast expresses predominantly Y₂ receptors, as shown by the opposite rank order of potency of NPY analogs, namely the high affinity of labeled and unlabeled PYY(3-36) but low affinity of [Leu³¹, Pro³⁴]-NPY and [Leu³¹, Pro³⁴]-PYY.

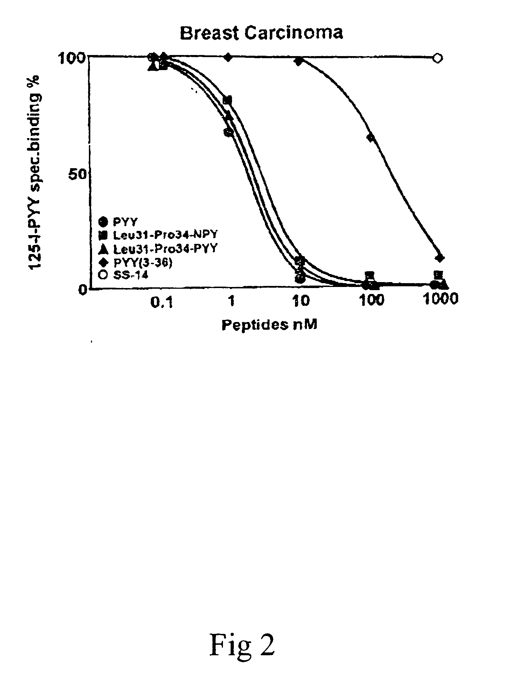

**Fig. 2** shows representative displacement curves using the universal ¹²⁵I-PYY radioligand and increasing concentrations of Y₁- and Y₂-selective analogs. While, in a typical Y₁-expressing breast tumor, the Y₁-selective [Leu³¹, Pro³⁴]-NPY and [Leu³¹, Pro³⁴]-PYY completely displace the radiotracer with high affinity, the Y₂-selective PYY(3-36) was inactive.

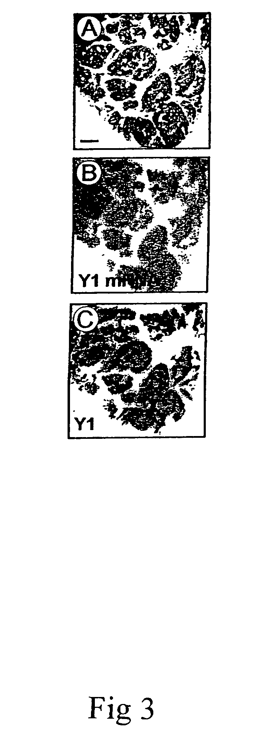

In situ hybridization for Y₁ and Y₂ mRNA was performed in cases selected for their high expression of the respective receptor proteins. Y₁ mRNA was consistently shown in the 12 investigated Y₁-type of tumors. Furthermore, it was possible to detect Y₂ mRNA in isolated Y₂-expressing tubules of the normal breast. **Fig. 3** illustrates Y₁ mRNA in a breast tumor.

### Discussion

This example is the first evidence that the neuropeptide NPY plays a potential role in cancer. It is remarkable that a great majority, i.e. 85% of human breast cancers, have an often high expression of NPY receptors. In all cases, the NPY receptor subtype Y₁ is expressed, whereas Y₂ is only expressed in 24% of the cases, and, when it is expressed, it never represents the predominant subtype of the tumor. In the 24% of the cases with a mixed expression of Y₁ and Y₂, a much more focal, topographically restricted distribution can be recognized for Y₂ than for Y₁, emphasizing once more the predominance of Y₁ in tumors. Both ductal and lobular breast cancers as well as all lymph node metastases can express NPY receptors.

Among the numerous cloned NPY receptors, the Y₁, Y₂, Y₄, and Y₅ represent at the moment the only fully defined subtypes (Michel et al. XVI. International union of pharmacology recommendations for the nomenclature of neuropeptide Y, peptide YY, and pancreatic polypeptide receptors. Pharmacol. Rev. 50, 143-150 (1998)). There are several arguments showing that the subtypes detected during the present study correspond to Y₁ and Y₂. Pharmacological evidence for Y₁ expression in tumors include a) specific binding of ¹²⁵I-PPY that is fully displaced in the high affinity range by the Y₁-selective [Leu³¹, Pro³⁴]-NPY, but not by PYY(3-36), PYY(13-36) or pancreatic polypeptide, compounds known to have high affinity for Y₂ or Y₄ (Michel et al. (1998), supra; Dumont et al. (1993), supra); b) selective binding of ¹²⁵I-[Leu³¹, Pro³⁴]-PYY in the same tissues (Gehlert & Gackenheimer (1997), supra); c) ionic, i.e. Ca⁺⁺-dependence typical for Y₁ (Wieland et al., Regul. Pept. 75-76, 263-269 (1998)); d) Y₁ mRNA detected by in situ hybridization in the tumor tissues. Furthermore, with those techniques we could confirm in humans the data from previous animal reports showing Y₁-expression in vessels (Bao et al., Proc. Natl. Acad. Sci. USA 94, 1261-1266 (1997); Hökfelt et al., Brain Res. Rev. 26, 154-166 (1998)).

## Claims

1. Use of a neuropeptide Y(NPY₁) receptor binding peptide compound, selected from the group consisting of [Leu³¹, Pro³⁴]-NPY, [Leu³¹, Pro³⁴]-PYY, Pro³⁴-PYY, NPY, PYY, Des Asn²⁹[Trp^{28,32}, Nva³⁴]-NPY(27-36), [Pro³⁰, Tyr³², Leu³⁴]-NPY(28-36), the dimer Bis (31/31'){[Cys³¹, Trp³², Nva³⁴]-NPY(31-36)}, SR120819A, BIBP3236, the compound 383U91 of the formula 
IleCysProCysTyrArgLeuArgTyrNH2
|          |
H2NTyrArgLeuArgTyrCysProCysIle,
 compound 1120W91 of the formula 
IleAsnProDpaTyrArgLeuArgTyrNH2
|
IleAsnProDpaTyrArgLeuArgTyrNH2,
 compound 1229U91 of the formula 
IleGluProDprTyrArgLeuArgTyrNH2
|   |
H2NTyrArgLeuArgTyrDprProGluIle
 for the preparation of a pharmaceutical composition for the diagnosis or treatment of tumors and their metastases overexpressing NPY₁ receptors, in particular breast cancer, ovarium cancer and glioblastoma, and their metastases.

2. The use as claimed in claim 1, wherein the peptide compound is provided with a label, selected from a radioactive metal isotope, a paramagnetic metal atom, and a radioactive halogen isotope.

3. The use as claimed in claim 2 for the preparation of a pharmaceutical composition for diagnosis, wherein the label is a radioactive metal isotope selected from the group consisting of ^{99m}Tc, ²⁰³Pb, ⁶⁷Ga, ⁶⁸Ga, ⁷²As, ¹¹¹In, ^{113m}In, ¹²³I, ¹⁷⁷Lu, ⁹⁷Ru, ⁶²Cu, ⁶⁴Cu, ⁵²Fe, ^{52m}Mn, and ⁵¹Cr.

4. The use as claimed in claim 2 for the preparation of a pharmaceutical composition for diagnosis, wherein the label is a paramagnetic metal atom selected from the group consisting of Cr, Mn, Fe, Co, Ni, Cu, Pr, Nd, Sm, Yb, Gd, Tb, Dy, Ho and Er.

5. The use as claimed in claim 2 for the preparation of a pharmaceutical composition for diagnosis, wherein the label is a radioactive halogen isotope selected from ¹²³I, ¹³¹I, ⁷⁵Br, ⁷⁶Br, ⁷⁷Br and ⁸²Br.

6. The use as claimed in claim 2, wherein the label is a therapeutical agent for the treatment of cancer, wherein the label is a radioisotope selected from the group consisting of ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I and ¹³¹I.

7. A pharmaceutical composition for the diagnosis of NPY₁ receptor overexpressing tumors, in particular breast cancer, ovarium cancer or glioblastoma, and their metastases, in particular in humans, comprising one or more peptide compounds, as defined in claim 1, and a suitable carrier, diluent and/or excipient, wherein the peptide compound(s) is/are labelled with (a) a radioactive metal isotope selected from the group consisting of ^{99m}Tc, ²⁰³Pb, ⁶⁷Ga, ⁶⁸Ga, ⁷²As, ¹¹¹In, ^{113m}In, ¹²³I, ¹⁷⁷Lu, ⁹⁷Ru, ⁶²Cu, ⁶⁴Cu, ⁵²Fe, ^{52m}Mn and ⁵¹Cr, or (b) a paramagnetic metal atom selected from the group consisting of Cr, Mn, Fe, Co, Ni, Cu, Pr, Nd, Sm, Yb, Gd, Tb, Dy, Ho and Er, or (c) a radioactive halogen isotope selected from ¹²³I, ¹³¹I, ⁷⁵Br, ⁷⁶Br, ⁷⁷Br and ⁸²Br.

8. A pharmaceutical composition for the treatment of NPY₁ receptor overexpressing tumors, in particular breast cancer, ovarium cancer or glioblastoma, and their metastases, in particular in humans, comprising one or more peptide compounds, as defined in claim 1, and a suitable carrier, diluent and/or excipient, wherein the peptide compound(s) is/are labelled with an isotope selected from the group consisting of ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ^{12I}Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd , ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I and ¹³¹I.

9. A kit for preparing a radiopharmaceutical composition, comprising (i) a NPY₁ receptor binding peptide compound, as defined in claim 1, which may be optionally derivatized by a reaction with a chelating agent and to which compound, if desired, an inert pharmaceutically acceptable carrier and/or formulating agents and/or adjuvants is/are added, (ii) a solution of a salt or chelate of a metal selected from the group consisting of the radioactive isotopes ²⁰³Pb, ⁶⁶Ga, ⁶⁷Ga, ⁶⁸Ga, ⁷²As, ¹¹¹In, ^{113m}In, ^{114m}In, ⁹⁷Ru, ⁶²Cu, ^{99m}Tc, ¹⁸⁶Re, ¹⁸⁸Re, ⁶⁴Cu, ⁵²Fe, ^{52m}Mn, ⁵¹Cr, ⁷⁷As, ⁹⁰Y, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁶⁶Ho, ¹⁷²Tm, ¹⁶⁹Yb, ¹⁷⁵Yb, ¹⁷⁷Lu, ¹⁰⁵Rh and ¹¹¹Ag, and (iii) instructions for use with a prescription for reacting the ingredients present in the kit.

10. A kit for preparing a radiopharmaceutical composition, comprising (i) a NPY₁ receptor binding peptide compound, as defined in claim 1, which may be optionally derivatized by a reaction with a chelating agent and to which compound, if desired, an inert pharmaceutically acceptable carrier and/or formulating agents and/or adjuvants is/are added, (ii) a reducing agent, and, if desired, a chelator, said ingredients (i) and (ii) optionally being combined, and (iii) instructions for use with a prescription for reacting the ingredients of the kit with ^{99m}Tc in the form of a pertechnetate solution or with ¹⁸⁶Re or ¹⁸⁸Re in the form of a perrhenate solution.

11. Use of a composition comprising, in a quantity sufficient for external imaging, a NPY₁ receptor binding peptide compound, as defined in claim 1, said compound being labelled with (a) a radioactive metal isotope selected from the group consisting of ^{99m}Tc, ²⁰³Pb, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn, and ⁵¹Cr, or (b) a paramagnetic metal atom selected from the group consisting of Cr, Mn, Fe, Co, Ni, Cu, Pr, Nd, Sm, Yb, Gd, Tb, Dy, Ho and Er, or (c) a radioactive halogen isotope, selected from ¹²³I, ¹³¹I, ⁷⁵Br, ⁷⁶Br, ⁷⁷Br and ⁸²Br, for the manufacture of a diagnostic agent for detecting and localizing NPY₁ receptor expressing tumors and their metastases in tissues, which in healthy condition do not contain NPY₁ receptor, in the body of a human being, by administering said composition to the being to be diagnosed in an amount *of 1 to 20 µg* by radioactive scanning or by magnetic resonance imaging, to determine the targeted sites in the body of said being in relation to the background activity, in order to allow detection and localization of the tumor(s) in the body.

12. Use of a composition comprising, in a quantity sufficient for detection by a gamma detecting probe, a NPY₁ receptor binding peptide compound, as defined in claim 1, said compound being labelled with a radioactive isotope selected from ¹⁶¹Tb, ¹²³I or ¹²⁵I, for the manufacture of a radioimmunodetection agent for intraoperatively detecting and localizing NPY₁ receptor expressing tumors and their metastases in tissues, which in healthy condition do not contain NPY₁ receptors, in the body of a human being, by administering said composition to the being to be diagnosed in an amount *of 1 to 20 µg* to determine the targeted sites in the body of said being, in order to allow detection and localization of the tumor(s) in the body.

13. Use of a composition comprising, in a quantity effective for combating or controlling tumors, a NPY1 receptor binding peptide compound, as defined in claim 1, said compound being labelled with an isotope selected from the group consisting of ¹⁸⁶Re, ¹⁸⁸Re, ¹⁷As, ⁹⁰Y, ⁶⁷Cu, ¹⁶Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹¹⁷Lu, ¹⁰⁵Rh, ¹¹¹Ag, ¹²⁴I and ¹³¹I, for the manufacture of a therapeutic agent for combating or controlling tumors and their metastases in tissues, which in healthy condition do not contain NPY₁ receptors, in the body of a human being, by administering said composition to the being to be treated in an amount *of 20 to 1000 µg.*

14. The use as claimed in any of claims 13-15, wherein the tumors to be detected, localized or therapeutically treated are selected from the group consisting of breast cancer, ovarian cancer and glioblastoma, and their metastases.

## Patentansprüche

1. Verwendung einer Neuropeptid Y₁ (NPY₁)-Rezeptor-bindenden-Peptidverbindung, ausgewählt aus der Gruppe, bestehend aus [Leu³¹, Pro³⁴]-NPY, [Leu³¹, Pro³⁴]-PYY, Pro³⁴-PYY, NPY, PYY, Des Asn²⁹[Trp^{28,32}, Nva³⁴]-NPY(27-36), [Pro³⁰, Tyr³², Leu³⁴]. NPY(28-36), dem Dimer Bis (31/31'){[Cys³¹, Trp³², Nva³⁴]-NPY(31-36)}, SR120819A, BIBP3236, der Verbindung 383U91 der Formel 
IleCysProCysTyrArgLeuArgTyrNH2
|     |
H2NTyrArgLeuArgTyrCysProCysIle,
 Verbindung 1120W91 der Fomel 
IleAsnProDpaTyrArgLeuArgTyrNH2
|
IleAsnProDpaTyrArgLeuArgTyrNH2,
 Verbindung 1229U91 der Formel 
IleGluProDprTyrArgLeuArgTyrNH2
|          |
H2NTyrArgLeuArgTyrDprProGluIle
 für die Präparation einer pharmazeutischen Zusammensetzung für die Diagnose oder die Behandlung von Tumoren und deren Metastasen, die NPY₁-Rezeptoren überexprimieren, insbesondere Brustkrebs, Eierstockkrebs und Glioblastom und deren Metastasen.

2. Verwendung, wie in Anspruch 1 beansprucht, wobei die Peptidverbindung mit einer Markierung zur Verfügung gestellt wird, die aus einem radioaktiven Metallisotop, einem paramagnetischen Metallatom und einem radioaktiven Halogenisotop ausgewählt ist.

3. Verwendung, wie in Anspruch 2 beansprucht, für die Präparation einer pharmazeutischen Zusammensetzung für die Diagnose, wobei die Markierung ein radiaktives Metallisotop ist, das ausgewählt ist aus der Gruppe, bestehend aus ^{99m}Tc, ²⁰³Pb, ⁶⁷Ga, ⁶⁸Ga, ⁷²As, ¹¹¹In, ^{113m}In, ¹²³I, ¹⁷⁷Lu, ⁹⁷Ru, ⁶²Cu, ⁶⁴Cu, ⁵²Fe, ^{52m}Mn und ⁵¹Cr.

4. Verwendung, wie in Anspruch 2 beansprucht, für die Präparation einer pharmazeutischen Zusammensetzung für die Diagnose, wobei die Markierung ein paramagnetisches Metallatom ist, das ausgewählt ist aus der Gruppe, bestehend aus Cr, Mn, Fe, Co, Ni, Cu, Pr, Nd, Sm, Yb, Gd, Tb, Dy, Ho und Er.

5. Verwendung, wie in Anspruch 2 beansprucht, für die Präparation einer pharmazeutischen Zusammensetzung für die Diagnose, wobei die Markierung ein radioaktives Halogenisotop ist, ausgewählt aus ¹²³I, ¹³¹I, ⁷⁵Br, ⁷⁶Br, ⁷⁷Br und ⁸²Br.

6. Verwendung, wie in Anspruch 2 beansprucht, wobei die Markierung ein therapeutisches Mittel für die Behandlung von Krebs ist, wobei die Markierung ein Radioisotop ist, ausgewählt aus der Gruppe, bestehend aus ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb ¹⁰⁹Pd, ¹⁶⁵Dy ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I und ¹³¹I.

7. Pharmazeutische Zusammensetzung für die Diagnose von NPY₁-Rezeptorüberexprimierenden Tumoren, insbesondere Brustkrebs, Eierstockkrebs oder Glioblastom und deren Metastasen, insbesondere in Menschen, umfassend eine oder mehrere Peptidverbindungen, wie in Anspruch 1 definiert, und einen geeigneten Träger, Verdünnungsmittel und/oder Hilfsstoff, wobei die Peptidverbindung(en) mit
(a) einem radioaktiven Metallisotop, ausgewählt aus der Gruppe, bestehend aus ^{99m}Tc, ²⁰³Pb, ⁶⁷Ga, ⁶⁸Ga, ⁷²As, ¹¹¹In, ^{113m}In, ¹²³I, ¹⁷⁷Lu, ⁹⁷Ru, ⁶²Cu, ⁶⁴Cu, ⁵²Fe, ^{52m}Mn, und ⁵¹Cr, oder
(b) einem paramagnetischen Metallatom, ausgewählt aus der Gruppe, bestehend aus Cr, Mn, Fe, Co, Ni, Cu, Pr, Nd, Sm, Yb, Gd, Tb, Dy, Ho und Er, oder
(c) einem radioaktiven Halogenisotop, ausgewählt aus ¹²³I, ¹³¹I, ⁷⁵Br, ⁷⁶Br, ⁷⁷Br und ⁸²Br, markiert ist/sind.

8. Pharmazeutische Zusammensetzung für die Behandlung von NPY₁-Rezeptorüberexprimierenden Tumoren, insbesondere Brustkrebs, Eierstockkrebs oder Glioblastom und deren Metastasen, insbesondere in Menschen, umfassend eine oder mehrere Peptidverbindungen, wie in Anspruch 1 definiert, und einen geeigneten Träger, Verdünnungsmittel und/oder Hilfsstoff, wobei die Peptidverbindung(en) mit einem Isotop, ausgewählt aus der Gruppe, bestehend aus ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I und ¹³¹I, markiert ist/sind.

9. Kit für die Herstellung einer radiopharmazeutischen Zusammensetzung, umfassend
i) eine NPY₁-Rezeptor-bindende Peptidverbindung, wie in Anspruch 1 definiert, welche optional durch eine Reaktion mit einem chelatbildenden Mittel derivatisiert sein kann, und zu welcher Verbindung, wenn gewünscht, ein inerter pharmazeutisch akzeptabler Träger und/oder formulierende Mittel und/oder Adjuvantien addiert wird/werden,
(ii) eine Lösung eines Salzes oder Chelat eines Metalls, ausgewählt aus der Gruppe, bestehend aus den radioaktiven Isotopen ²⁰³Pb, ⁶⁶Ga, ⁶⁷Ga, ⁶⁸Ga, ⁷²As, ¹¹¹In, ^{113m}In, ^{114m}In, ⁹⁷Ru, ⁶²Cu, ^{99m}Tc, ¹⁸⁶Re, ¹⁸⁸Re, ⁶⁴Cu, ⁵²Fe, ^{52m}Mn, ⁵¹Cr, ⁷⁷AS, ⁹⁰Y, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁶⁶Ho, ¹⁷²Tm, ¹⁶⁹Yb, ¹⁷⁵Yb, ¹⁷⁷Lu, ¹⁰⁵Rh und ¹¹¹Ag, und
(iii) Instruktionen für die Verwendung mit einer Verschreibung für das Reagieren der Inhaltsstoffe, die in dem Kit vorhanden sind.

10. Kit für die Herstellung einer radiopharmazeutischen Zusammensetzung, umfassend
(i) eine NPY₁-Rezeptor-bindende Peptidverbindung, wie in Anspruch 1 definiert, welche optional durch eine Reaktion mit einem chelatbildenden Mittel derivatisiert sein kann, und zu welcher Verbindung, wenn gewünscht, ein inerter pharmazeutisch akzeptabler Träger und/oder formulierende Mittel und/oder Adjuvantien addiert wird/werden,
(ii) ein reduzierendes Mittel und, wenn gewünscht, ein Chelator,
wobei die Inhaltsstoffe (i) und (ii) optional kombiniert werden, und
(iii) Instruktionen für die Verwendung mit einer Verschreibung für das Reagieren der Inhaltsstoffe des Kits mit ^{99m}Tc in Form einer Perrtechnetat-Lösung oder mit ¹⁸⁶Re oder ¹⁸⁸Re in Form einer Perrhenat-Lösung.

11. Verwendung einer Zusammensetzung, umfassend in einer Quantität, die ausreichend für externe Bildgebung ist, eine NPY₁-Rezeptor-bindende Peptidverbindung, wie in Anspruch 1 definiert, wobei die Verbindung markiert ist mit
(a) einem radioaktiven Metallisotop, ausgewählt aus der Gruppe, bestehend aus ^{99m}Tc, ²⁰³Pb, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn und ⁵¹Cr, oder
(b) einem paramagnetischen Metallatom, ausgewählt aus der Gruppe, bestehend aus Cr, Mn, Fe, Co, Ni, Cu, Pr, Nd, Sm, Yb, Gd, Tb, Dy, Ho und Er, oder
(c) einem radioaktivem Halogenisotop, ausgewählt aus ¹²³I, ¹³¹I, ⁷⁵Br, ⁷⁶Br, ⁷⁷Br und ⁸²Br,
für die Herstellung eines diagnostischen Mittels für die Detektion und Lokalisierung von NPY₁-Rezeptor-exprimierenden Tumoren und deren Metastasen in Geweben, welche unter gesunden Bedingungen keine NPY₁-Rezeptoren enthalten, im Körper eines Menschen,
durch Verabreichen der Zusammensetzung an den Menschen, der diagnostiziert werden soll, in einer Menge *von 1 bis 20 µg*
durch radioaktive Abtastung oder durch bildgebende Kernspintomographie, um die gezielten Stellen in dem Körper des Menschen in Bezug auf die Hintergrundaktivität zu bestimmen, um Detektion und Lokalisierung des Tumors /der Tumoren in dem Körper zu erlauben.

12. Verwendung einer Zusammensetzung, umfassend in einer Quantität, die ausreichend für die Detektion einer gamma-detektierenden Probe ist, eine NPY₁-Rezeptor-bindende Peptidverbindung, wie in Anspruch 1 definiert, wobei die Verbindung mit einem radioaktiven Isotop, ausgewählt aus ¹⁶¹Tb, ¹²³I oder ¹²⁵I, markiert ist,
für die Herstellung eines Radioimmunodetektions-Mittels für das intraoperative Detektieren und Lokalisieren von NPY₁-Rezeptor-exprimierenden Tumoren und deren Metastasen in Geweben, welche unter gesunden Bedingungen keine NPY₁-Rezeptoren enthalten, im Körper eines Menschen,
durch Verabreichen der Zusammensetzung an den Menschen, der diagnostiziert werden soll, in einer Menge *von 1 bis 20 µg,* um die gezielten Stellen des Körpers des Menschen zu bestimmen, um Detektion und Lokalisierung des Tumors/der Tumoren in dem Körper zu erlauben.

13. Verwendung einer Zusammensetzung, umfassend in einer Quantität, die für die Bekämpfung oder die Kontrolle von Tumoren wirksam ist, eine NPY₁-Rezeptor-bindende Peptidverbindung, wie in Anspruch 1 definiert, wobei die Verbindung mit einem Isotop markiert ist, ausgewählt aus der Gruppe, bestehend aus ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I und ¹³¹I, für die Herstellung eines therapeutischen Mittels für die Bekämpfung oder Kontrolle von Tumoren und deren Metastasen in Geweben, welche unter gesunden Bedingungen keine NPY₁-Rezeptoren enthalten, im Körper eines Menschen,
durch Verabreichen der Zusammensetzung an den Menschen, der behandelt werden soll, in einer Menge *von 20 bis 1000,ug.*

14. Verwendung, wie in einem der Ansprüche 13 bis 15 beansprucht, wobei die Tumoren, die detektiert, lokalisiert oder therapeutisch behandelt werden sollen, ausgewählt sind aus der Gruppe, bestehend aus Brustkrebs, Eierstockkrebs und Glioblastom und deren Metastasen.

## Revendications

1. Application d'un composé de peptide de liaison de récepteur de neuropeptide Y₁ (NPY₁), choisi dans le groupe constitué par [Leu³¹, Pro³⁴] -NPY, [Leu³¹, Pro³⁴] -PYY, Pro³⁴-PYY, NPY, PYY, DesAsn²⁹ [Trp^{28,32}, Nva³⁴]-NPY (27-36), [Pro³⁰, Tyr³², Leu³⁴) - NPY (28-36) , le dimère Bis (31/31') {[Cys³¹ ,Trp³² ,Nva³⁴] -NPY (31-36) } , SR120819A, BIBP3236,
le composé 383U91 de formule 
IleCysProCysTyrArgLeuArgTyrNH2
|          |
H2NTyrArgLeuArgTyrCysProCysIIe,
 le composé 1120W91 de formule 
IleAsnProDpaTyrArgLeuArgTyrNH2
|
IleAsnProDpaTyrArgLeuArgTyrNH2,
 le composé 1229U91 de formule 
IleGluProDprTyrArgLeuArgTyrNH2
|     |
H2NTyrArgLeuArgTyrDprProGluIle
 à la préparation d'une composition pharmaceutique pour le diagnostic ou le traitement des tumeurs et de leurs métastases surexprimant des récepteurs de NPY₁, en particulier le cancer du sein, le cancer des ovaires et le glioblastome, et leurs métastases.

2. Application selon la revendication 1, dans laquelle le composé de peptide comporte un marqueur choisi parmi un isotope métallique radioactif, un atome métallique paramagnétique, et un isotope d'halogène radioactif.

3. Application selon la revendication 2 à la préparation d'une composition pharmaceutique pour le diagnostic, dans laquelle le marqueur est un isotope métallique radioactif, choisi dans le groupe constitué par ^{99m}Tc, ²⁰³Pb, ⁶⁷Ga, ⁶⁸Ga, ⁷²As, ¹¹¹In, ^{113m}In, ¹²³I , ¹⁷⁷ Lu, ⁹⁷Ru, ⁶²Cu, ⁶⁴Cu, ⁵²Fe , ^{52m}Mn et ⁵¹Cr.

4. Application selon la revendication 2 à la préparation d'une composition pharmaceutique pour le diagnostic, dans laquelle le marqueur est un atome métallique paramagnétique choisi dans le groupe constitué par Cr, Mn, Fe, Co, Ni, Cu, Pr, Nd, Sm, Yb, Gd, Tb, Dy, Ho et Er.

5. Application selon la revendication 2 à la préparation d'une composition pharmaceutique pour le diagnostic, dans laquelle le marqueur est un isotope d'halogène radioactif choisi parmi ¹²³I, ¹³¹I, ⁷⁵Br, ⁷⁶Br, ⁷⁷Br et ⁸²Br.

6. Application selon la revendication 2, dans laquelle la marqueur est un agent thérapeutique pour le traitement du cancer, et dans laquelle le marqueur est un radio-isotope choisi dans le groupe constitué par ^{114m}In, ¹⁸⁶Re, ¹⁸⁸Re, ⁷⁷As**,** ⁹⁰Y, ⁶⁶Ga, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd**,** ¹⁵⁹Gd, ¹⁶⁶Ho**,** ¹⁷²Tm, ¹⁶⁹Yb, ¹⁷⁵Yb, ¹⁷⁷Lu, ¹⁰⁵Rh, ¹¹¹Ag, ¹²⁴I et ¹³¹**I** .

7. Composition pharmaceutique pour le diagnostic des tumeurs surexprimant des récepteurs de NPY₁, en particulier le cancer du sein, le cancer des ovaires et le glioblastome, et leurs métastases, en particulier chez les êtres humains, comprenant un ou plusieurs composés de peptides tels que définis par la revendication 1, et un véhicule, diluant et/ou excipient convenable, dans laquelle le composé ou les composés de peptides sont marqués par (a) un isotope métallique radioactif, choisi dans le groupe constitué par ^{99m}Tc, ²⁰³Pb, ⁶⁷Ga , ⁶⁸Ga, ⁷²As, , ¹¹¹In , ^{113m}In , ¹²³I , ¹⁷⁷Lu, , ⁹⁷Ru , ⁶²Cu, ⁶⁴Cu , ⁵²Fe, ^{52m}Mn et ⁵¹Cr, ou (b) un atome métallique paramagnétique choisi dans le groupe constitué par Cr, Mn, Fe, Co, Ni, Cu, Pr, Nd, Sm, Yb, Gd, Tb, Dy, Ho et Er, ou (c) un isotope d'halogène radioactif choisi parmi ¹²³I, ¹³¹I, ⁷⁵Br, ⁷⁶Br, ⁷⁷Br et ⁸²Br.

8. Composition pharmaceutique pour le diagnostic des tumeurs surexprimant des récepteurs de NPY₁, en particulier le cancer du sein, le cancer des ovaires et le glioblastome, et leurs métastases, en particulier chez les êtres humains, comprenant un ou plusieurs composés de peptides tels que définis par la revendication 1, et un véhicule, diluant et/ou excipient convenable, dans laquelle le composé ou les composés de peptides sont marqués par un isotope choisi dans le groupe constitué par ^{114m}In, ¹⁸⁶Re, ¹⁸⁸Re, ⁷⁷As, ⁹⁰Y, ⁶⁶Ga, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I et ¹³¹I.

9. Kit pour la préparation d'une composition radio-pharmaceutique, comprenant (i) un composé de peptide de liaison de récepteur de neuropeptide NPY₁, tel que défini par la revendication 1, qui peut éventuellement être dérivé par réaction avec un agent chélatant et auquel composé peuvent être ajoutés, le cas échéant, un véhicule et/ou des agents de formulation et/ou des adjuvants inertes et acceptables pharmaceutiquement, (ii) une solution d'un sel ou d'un chélate d'un métal choisi dans le groupe constitué des isotopes radioactifs ²⁰³Pb, ⁶⁶Ga, ⁶⁷Ga, ⁶⁸Ga, ⁷²As, ¹¹¹In, ^{113m} In, ^{114m}In, ⁹⁷Ru, ⁶²Cu, ^{99m}Tc, ¹⁸⁶Re, ¹⁸⁸Re, ⁶⁴Cu, ⁵²Fe, ^{52m}Mn, ⁵¹Cr, ⁷⁷As, ⁹⁰Y, ⁶⁷Cu, ¹⁶⁹Er, ^{117m}Sn, ¹²¹Sn, ¹²⁷Te, ¹⁴²Pr, ¹⁴³Pr, ¹⁹⁸Au, ¹⁹⁹Au, ¹⁴⁹Tb, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁶⁶Ho, ¹⁷²Tm, ¹⁶⁹Yb, ¹⁷⁵Yb, ¹⁷⁷Lu, ¹⁰⁵Rh et ¹¹¹Ag, et (iii) des instructions d'utilisation avec une prescription pour la réaction des ingrédients présents dans le kit.

10. Kit pour la préparation d'une composition radio-pharmaceutique, comprenant (i) un composé de peptide de liaison de récepteur de neuropeptide NPY₁, tel que défini par la revendication 1, qui peut éventuellement être dérivé par réaction avec un agent chélatant et auquel composé peuvent être ajoutés, le cas échéant, un véhicule et/ou des agents de formulation et/ou des adjuvants inertes et acceptables pharmaceutiquement, (ii) un agent réducteur et, le cas échéant, un agent de chélation, les ingrédients (i) and (ii) étant éventuellement combinés, et (iii) des instructions d'utilisation avec une prescription pour la réaction des ingrédients présents dans le kit avec ^{99m}Tc sous forme d'une solution de pertechnétate ou avec 186Re ou 188Re sous forme d'une solution de perrhénate.

11. Application d'une composition contenant, en quantité suffisante pour la formation d'un image externe, un composé de peptide de liaison de récepteur de neuropeptide NPY₁, tel que défini par la revendication 1, le composé étant marqué par (a) un isotope métallique radioactif, choisi dans le groupe constitué par ^{99m}Tc, ²⁰³Pb, ⁶⁷Ga, ⁶⁸Ga, ⁷²As, ¹¹¹In, ^{113m}In, ⁹⁷Ru, ⁶²Cu, ⁶⁴Cu, ⁵²Fe, ^{52m}Mn et ⁵¹Cr, ou (b) un atome métallique paramagnétique choisi dans le groupe constitué par Cr, Mn, Fe, Co, Ni, Cu, Pr, Nd, Sm, Yb, Gd, Tb, Dy, Ho et Er, ou (c) un isotope d'halogène radioactif choisi parmi ¹²³I, ¹³¹I, ⁷⁵Br, ⁷⁶Br, ⁷⁷Br et ⁸²Br, à la fabrication d'un agent de diagnostic pour la détection et la localisation de tumeurs exprimant des récepteurs de NPY₁ et de leurs métastases dans des tissus qui, dans des conditions saines, ne contiennent pas de récepteurs de NPY₁, dans le corps d'un être humain, par administration de la composition à l'être qui doit subir le diagnostic en quantité comprise entre 1 et 20 µg par imagerie par résonance magnétique ou par balayage radioactif, pour la détermination de sites ciblés dans le corps de l'être en relation avec l'activité du fond continu, afin que la tumeur ou les tumeurs puissent être détectées et localisées dans le corps.

12. Application d'une composition contenant, en quantité suffisante pour la détection par une sonde de détection gamma, un composé de peptide de liaison de récepteur de neuropeptide NPY₁, tel que défini par la revendication 1, le composé étant marqué par un isotope radioactif choisi parmi ¹⁶¹Tb, ¹²³I et ¹²⁵I, à la fabrication d'un agent de radioimmuno-détection pour la détection et la localisation intraopératoire de tumeurs exprimant des récepteurs de NPY₁ et de leurs métastases dans des tissus qui, dans des conditions saines, ne contiennent pas de récepteurs de NPY₁, dans le corps d'un être humain, par administration de la composition à l'être qui doit subir le diagnostic en quantité comprise entre 1 et 20 µg pour la détermination de sites ciblés dans le corps de l'être, afin que la tumeur ou les tumeurs puissent être détectées et localisées dans le corps.

13. Application d'une composition contenant, en quantité efficace pour combattre ou maîtriser des tumeurs, un composé de peptide de liaison de récepteur de neuropeptide NPY₁, tel que défini par la revendication 1, le composé étant marqué par un isotope choisi dans le groupe constitué par ¹⁸⁶Re, ¹⁸⁸Re, ⁷⁷As, ⁹⁰Y, ⁶⁷Cu, ¹⁶⁹Er, ¹²¹Sn, ¹²⁷Te, ¹⁴²Pr, ¹⁴³Pr, ¹⁹⁸Au, ¹⁹⁹Au, ¹⁶¹Tb, ¹⁰⁹Pd, ¹⁶⁵Dy, ¹⁴⁹Pm, ¹⁵¹Pm, ¹⁵³Sm, ¹⁵⁷Gd, ¹⁵⁹Gd, ¹⁶⁶Ho, ¹⁷²Tm, ¹⁶⁹Yb, ¹⁷⁵Yb, ¹⁷⁷Lu, ¹⁰⁵Rh, ¹¹¹Ag, ¹²⁴I et ¹³¹I, à la fabrication d'un agent thérapeutique pour combattre ou maîtriser des tumeurs et leurs métastases dans des tissus qui, dans des conditions saines, ne contiennent pas de récepteurs de NPY₁, dans le corps d'un être humain, par administration de la composition à l'être afin qu'il soit traité par une quantité de 20 à 1 000 µg.

14. Application selon l'une quelconque des revendications 13 à 15, dans laquelle les tumeurs à détecter, localiser ou traiter thérapeutiquement sont choisies dans le groupe qui est constitué par le cancer du sein, le cancer des ovaires et le glioblastome, et leurs métastases.
